**Europäisches Patentamt**

**European Patent Office**

**Office européen des brevets**

(19)

(11) Publication number: **0 210 969**
**A2**

(12) ## EUROPEAN PATENT APPLICATION

(21) Application number: **86850255.0**

(22) Date of filing: **15.07.86**

(51) Int. Cl.⁴: **A 61 F 13/18**

(30) Priority: **31.07.85 JP 169631/85**

(43) Date of publication of application:
**04.02.87 Bulletin 87/6**

(84) Designated Contracting States:
**BE DE FR NL SE**

(71) Applicant: **Mölnlycke AB**
**S-405 03 Göteborg(SE)**

(72) Inventor: **Boman, Lars**
**Krokslätts Parkgata 5B**
**S-43138 Mölndal(SE)**

(74) Representative: **Alfredson, Stig et al,**
**H. ALBIHNS PATENTBYRA AB Box 7664**
**S-103 94 Stockholm(SE)**

(54) Absorbent product such as an absorption body for diapers or sanitary towels, and a method of producing said product.

(57) The invention relates to an absorbent product, such as an absorption body for a diaper or sanitary towel, and a method of producing such a product.

The object is to reduce leakage round the edges of the product, and according to the invention this is achieved by having regions or strings (2, 3) of high-absorption material arranged along the edges of the body (1), these regions or strings giving a barrier effect against liquid migration. Liquid distribution in the high-absorption material may be improved and the momentary concentration of liquid in certain areas may be evened out by providing longitudinal compression lines (4,5) in the product, inwards of the regions of high-absorption material. Transverse compression lines (6-14) may also be arranged optionally between the longitudinal lines.

A more rapid evening-out of liquid is obtained by interrupting the longitudinal compression lines.

FIG.3

EP 0 210 969 A2

0210969

Absorbent product such as an absorption body for diapers or sanitary towels, and a method of producing said product

The present invention relates to absorbent products in the form of an absorption body for diapers or sanitary towels and is intended to provide an improved moisture seal at the edges of the product, as well as increased capacity for taking up liquids.

The problems, particularly with diapers, are inter alia leakage along the side edges, especially when the diaper has been worn too long.

The problem mentioned has been surmounted by the present invention, by a high-absorption material being inserted, at least in the edge regions of the product, the material being fastened to the diaper absorption body with hot-melt glue. By using a very small amount of glue, absorption is not affected since only small portions of the particles in the high-absorption material are affected by the glue, although the latter fastens the material very well, the diaper sheath enveloping the absorption body also keeping the high-absorption material in place.

A region or string with high-absorption material functions such that it has a greater capacity for taking up liquid than the surrounding absorption body, and such that it does not give up absorbed liquid to its surroundings. There is thus formed an outward barrier layer, which also increases the ability of the absorbent product for taking up water. The favourable action of the high-absorption may be still further improved by placing a compression line, known per se, inwards of the region or string of this material.

The use of high-absorption material in disposible products has already been proposed per se. However, the problem has been that the powdery material has not been able to be fixed satisfactorily. This problem has been solved by the present invention, while there has been obtained at the same time the possibility of readily locating the high-absorption material optionally on the absorption body.

The invention will now be described in detail in the following, with reference to the accompanying drawing, where

Figure 1 is a plan of an absorption body for a diaper in accordance with the invention,

Figure 2 is a section along the line II-II in Fig 1, and

Figure 3 finally illustrates another embodiment of the absorption body made in accordance with the invention.

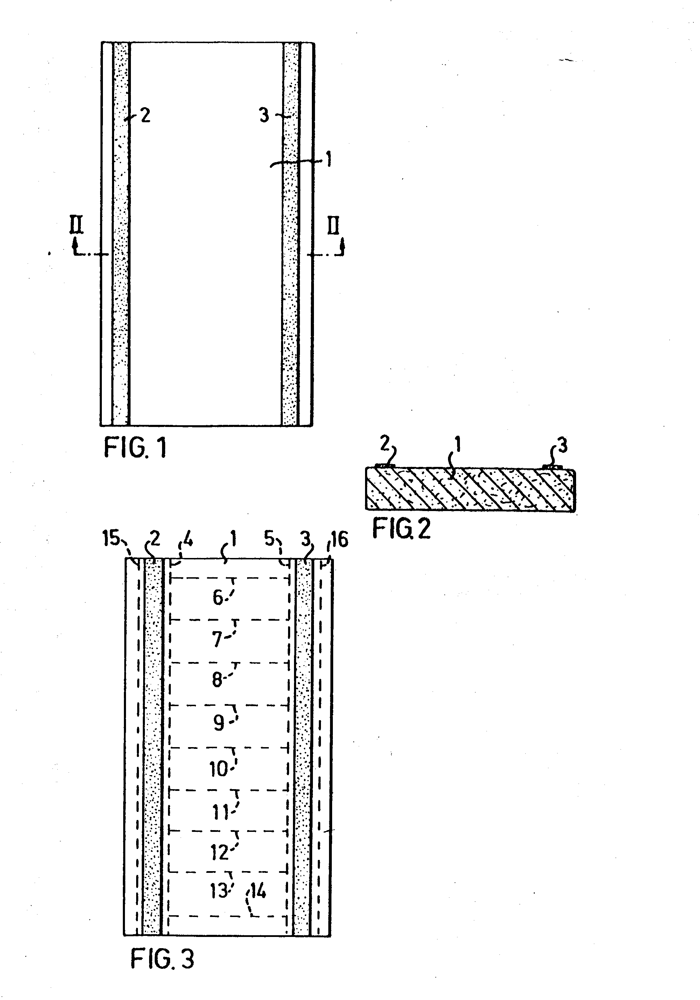

In Fig 1 there is illustrated an absorption body 1, implemented in accordance with the invention. This body is illustrated without its sheath, better to illustrate regions or strings 2,3 where high-absorption material has been placed. As already mentioned, this material, which is usually available in powder form, is fixed in place with the aid of a very thin non-covering coating of hot-melt glue.

This glue may be applied as a spray at the present time. To fix the high-absorption powder, the glue is suitably applied by spraying it in a network-like pattern in accordance with the invention. The powder is strewn on to the hot-melt coating, which gives a comparatively sparse powder deposit, enabling the swelling capacity of the high-absorption material to be utilized to the full.

Since the high-absorption powder grains are only affected in patches by the glue, their capacity for taking up liquid is not deteriorated. The barrier effect caused by the use of the high-absorption material is due to the spread of liquid in this material being poorer than in the rest of the absorption body. The transport of liquid from absorption body to high-absorption material is facilitated by the difference in capillary action, while migration of liquid in the opposite direction is made more difficult.

There may be a temporary uneven distribution of liquid in the high-absorption material, and to remedy this, as well as to increase the capacity for taking up liquid, a line 4,5 may to advantage be compressed into the absorption body 1 inwards of each region 2,3 of high-absorption material. In this way the longitudinal spread of liquid in the diaper will be improved considerably, and it will also be taken up more rapidly (see Fig 2). The compression lines may also be interrupted to provide balanced compression liquid distribution in the high-absorption material.

Even more rapid take-up of liquid may be achieved by arranging transverse compression lines 6-14 on the absorption body between the longitudinal compression lines 4,5 (see Fig 3). The two outermost lines 6 and 14 then serve as barriers in the longitudinal direction of the diaper. These two lines may be replaced by strings of high-absorption material, or be supplemented by such material, but this has not been found necessary as a rule. As a further precaution, compression lines 15,16 may be arranged outwards of the regions or strings 2,3 of high-absorption material, but this is also an unnecessary measure for normal uses.

With a diaper implemented in accordance with the invention, there is obtained at low cost a considerable reduction in the risk of leakage along the edges of the diaper as well as improved absorption capacity.

The invention is not restricted to the embodiments described above, since a plurality of modifications are possible within the scope of the following claims.

In the embodiments described hereinbefore, the regions or strings 2,3 of high-absorption material have been arranged along the edges of an absorption body on the side thereof facing away from the wearer when the product is in use. This barrier effect against lateral leakage, achieved by the regions or strings 2,3, may be considerably reinforced if the high-absorption powder is disposed all the way round the edges of the absorption body.

With the help of hot-melt spray, high-absorption powder may be applied to optional locations on an absorption body. For example, with an absorption core having folded-in portions of a material web intended to form the core, where the web is provided with folding lines allowing the formation of strips extending along these lines, the web may have a deposit of high-absorption powder which serves as protection against edge leakage after folding.

CLAIMS

1. Absorbent product such as the absorption body for a diaper or sanitary towel, characterized in that there are arranged regions or strings (2,3) of high-absorption material along the edges of the absorption body (1), and also optional compression lines (4,5; 6-14,15,16) for obtaining improved sealing against leakage round the product.

2. Product as claimed in claim 1, characterized in that the longitudinal compression lines (4,5) are interrupted in one or more places.

3. Product as claimed in claim 1 or 2, characterized in that inwards of the regions or strings (2,3) of high-absorption material there are arranged longitudinal compression lines (4,5).

4. Product as claimed in any one of claims 1-3, characterized in that transverse compression lines (6-14) are arranged between longitudinal compression lines (4,5).

5. Product as claimed in any one of claims 1-4, characterized in that the outmost transverse lines (6,14) comprise high-absorption material.

6. Method of producing an absorbent product such as an absorption body for diapers or sanitary towels, for achieving increased resistance to leakage round the product, characterized in that regions or strings of high-absorption material are arranged along the edges of the product.

7. Method as claimed in claim 6, characterized in that the high-absorption material is fixed to the product with the aid of hot-melt glue spraying.

FIG. 1

FIG. 2

FIG. 3